# EUROPEAN PATENT APPLICATION

(11) **EP 1 462 103 A1**
(43) Date of publication of application: **29.09.2004**
(21) Application number: 04290804.6
(22) Date of filing: 25.03.2004
(51) Int. Cl.: A61K 31/403, A61K 31/437, C07D 209/86, C07D 401/04, A61P 25/00

(54) **NO donors, combination products and uses as modulators of neurotransmitter release**

(30) Priority: 25.03.2003 EP 03360042
(71) Applicant: Faust Pharmaceuticals, 67400 Illkirch (FR)
(72) Inventor: Schann, Stephan, 67000 Strasbourg (FR); Neuville, Pascal, 67610 La Wantzenau (FR)
(74) Representative: Grosset-Fournier, Chantal Catherine

(57) **Abstract**

The present invention relates to compounds of the general formula (I) or (II): 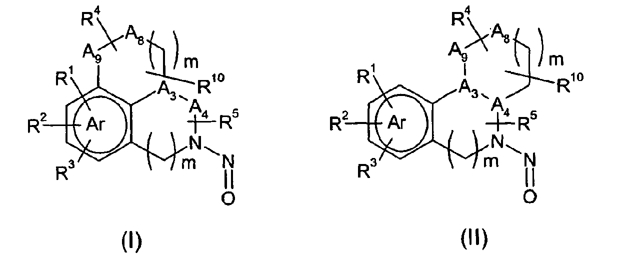 wherein:
**R**^{**1**}**, R**^{**2**}**, R**^{**3**}**, R**^{**4**}**, R**^{**5**} and **R**^{**10**} are, independently from one another, H or a moiety of the following general formula : **-(R**^{**6**}**)**_{**n**}**-R**^{**7**}, with **R**^{**6**} is an alkyl chain and **R**^{**7**} is, a moiety selected in the group consisting of -C_{n'}H_{2n'+1}, a cycloalkyl moiety, -N(C_{n'}H_{2n'+1})(C_{n'}H_{2n'+1}), -NH-cycloalkyl, -O(C_{n'}H_{2n'+1}), -O-cycloalkyl, =O, =S, -NO₂, , -I, -Br, -Cl, -F, -CF₃, -OCF₃, -COOH, -SO₃H, -PO₃H₂, -CN, **A**_{**3**}**, A**_{**4**}**, A**_{**8**} and **A**_{**9**} are C, N, O and S, **m** is 0 to 2, **n** is 0 to 6, and to their use for the treatment and/or prevention of diseases and conditions mediated by the imbalance of acetylcholine, and for treating and/or preventing glutamate excitotoxicity.

## Description

The present invention relates to compounds, combination products, compositions including them and methods for controlling neurotransmitters levels in mammals using these compounds, products or compositions. It further concerns methods for treating and/or preventing various diseases or pathological conditions which are associated with neurological dysfunctions caused by alteration in neurotransmitter levels, especially in the nervous tissues. According to particular aspects, the present invention relates to compounds, combination products, compositions and methods useful for controlling acetylcholine levels and for treating and/or preventing various diseases and conditions mediated by the imbalance of said neurotransmitter. The properties of the compounds, combination products and compositions of the invention make them particularly useful in the treatment and/or prevention, for example, of cognitive disorders and/or neurological dysfunction and/or mood disturbances such as, but not limited to, degenerative nervous system diseases or dementia. Additionally, these combination products can be used as reagents in studies on the biochemical mechanism of neurotransmitter based diseases.

The following description is provided to aid in understanding the invention but is not admitted to be prior art to the invention.

Appropriate intercellular signals between neurons are required for normal neurological functions. Neuronal signals are transmitted from cell to cell at specialized sites of contact known as synapses. The usual mechanism of transmission is indirect. The cells are electrically isolated from one another, the presynaptic cell being separated from the postsynaptic cell by a narrow synaptic cleft. A change of electrical potential in the presynaptic cell triggers it to release signalling chemical molecules known as neurotransmitters. The neurotransmitters rapidly diffuse across the synaptic cleft and provoke an electrical change in the postsynaptic cell by binding to neurotransmitter specific receptors leading to ion exchanges throughout ion channels. After release, the excess neurotransmitters are rapidly removed, either by specific enzymes in the synaptic cleft or by reuptake into the presynaptic cell or surrounding glial cells. Reuptake is mediated by a variety of neurotransmitter transporters. Rapid removal ensures both spatial and temporal precision of signalling at a synapse. For example, rapid reuptake can prevent excess neurotransmitters from influencing neighbouring cells and can clear the synaptic cleft before the next pulse of neurotransmitter release so that the timing of repeated, rapid signalling events is accurately communicated to the postsynaptic cell. This tight intercellular communication is disrupted when an imbalance of neurotransmitters occurs (e.g. when not enough neurotransmitter is synthesized and/or released from presynaptic cells or the reuptake of neurotransmitters by presynaptic cells is too rapid) leading thereby to neurological dysfunctions.

Neurotransmitters are numerous, with new candidates being discovered continuously. The main neurotransmitters are acetylcholine (ACh), norepinephrine (NE), gamma-aminobutyric acid (GABA), dopamine (DO), serotonin (S), glutamate (Glu), and nitric oxide (NO). Literature provides numerous examples illustrating that neurotransmitters are closely involved in a wide array of diseases or pathological conditions which are associated with neurological dysfunctions caused by, for example, alteration in neurotransmitter levels and/or their ratio levels. Many of these diseases correlate with increasing age. Although in early stages of some diseases certain systems are rather specifically affected (e.g. the cholinergic system in Alzheimer's Disease (AD) and Myasthenia Gravis ; the dopaminergic system in Parkinson's Disease, etc.), multiple neurotransmitter system deficiencies (acetylcholine, dopamine, norepinephrine, serotonin) are generally found at later stages of disease such as senile dementia, multi-infarct dementia, Huntington's disease, mental retardation, etc..., leading to multiple symptoms that include cognitive, neurological, and effective/psychotic components.

Acetylcholine (ACh) is one of the major modulators of brain functions and it is the main neurotransmitter at the peripheral nervous system. Modulation of acetylcholine levels is thereby crucial for nervous system function. It is synthesized and released at the cholinergic nerve terminals by the enzyme choline acetyltransferase (ChAT), is accumulated in synaptic vesicles by the activity of a vesicular acetylcholine transporter (VAChT) and is hydrolyzed by acetylcholinesterase AChE) or butyrylcholinesterase (BuChE).

Alzheimer disease (AD) is a neurodegenerative condition leading to progressive, irreversible loss of cognitive and behavioural function which is characterized by a progressive loss of memory and inability to carry out normal activities of daily living and is frequently accompanied by changes in behaviour and personality. Alzheimer's disease is associated with degeneration of central cholinergic neurons (Coyle et al., 1983, Science, 219, 1184-1190). Moreover, in addition to significant neuronal cell loss, there is now long standing evidence suggesting that the neuropsychiatric manifestations of AD are implicating cholinergic system deficits such as, for example, decreases or deficiencies in choline acetyltransferase (ChAT) activity (Lehericy et al., 1993, J. Comp. Neurol., 330, 15-31), high affinity choline uptake (Rylett et al., 1983, Brain Res., 289, 169-175), acetylcholine (ACh) release (Nilsson et al., 1986, J. Neural Transm., 67, 275-285), or both nicotinic and muscarinic receptor binding property (Perry et al., 1995, Neuroscience, 64 , 385-395). These cholinergic deficits positively correlate with cognitive impairments in AD (DeKosky et al., 1992, Ann. Neurol., 32, 625-632), as well as with non-cognitive behavioural disturbances (Minger et al., 2000, Neurology, 55, 1460-1467). Similarly, several types of dementia (e.g. AD related dementia, dementia with Lewy bodies, Parkinson's disease dementia or vascular dementia subtypes) associated with multiple behavioural and psychological symptoms such as aggressive behaviour, psychosis, apathy, anxiety, depression, hallucinations, delusions and overactivity have also been shown to be associated with disturbance of the cholinergic systems (Minger et al., *supra).*

Therapeutic approaches have been developed to address the clinical aspects of acetylcholine neurotransmission dysfunctions. The different approaches have focused on the following strategies:
(i) provision of additional substrate for synthesis of acetylcholine (e.g., choline, CDP-choline, phosphatidyl choline,...);
(ii) stimulation of production and secretion of acetylcholine (e.g., phosphatidyl serine, CDP-choline, huperzine A ...);
(iii) increase of the synaptic availability of acetylcholine by inhibiting acetylcholinesterase/ butyrylcholinesterase (AChE/BuChE) [e.g., tacrine (Cognex), donepezil (Aricept), galantamine (Reminyl), rivastigmine (Exelon), huperzine A];
(iv) inhibition of acetylcholine re-uptake ;
(v) provision of agents that facilitate improved binding at the receptor site (e.g., phosphatidyl serine and dioleylphosphatidic acid);
(vi) mimicking the effects of acetylcholine by acting directly on, for example, nicotinic receptors (nicotinic agonists, e.g. ABT-594); and
(vii) induction of enzymes used to synthesize acetylcholine (e.g., soy phytoestrogens).

However, despite these considerable investments in neuroscience research, only four drugs, all cholinesterase inhibitors, have actually been approved for the symptomatic management of AD in the United States. Although basically safe and modestly effective, these drugs are far from ideal, being neither universally efficacious nor disease modifying.

Thus, there is still a need for developing new compounds which address the neurotransmission dysfunctions and associated clinical aspects thereof, and more precisely the acetylcholine neurotransmission dysfunctions and associated clinical aspects thereof, by controlling the acetylcholine levels in a patient, especially in nervous tissue (including both the Central Nervous System, or CNS, and the Peripheral Nervous System, or PNS).

The Inventors have now identified novel compounds having regulatory activities towards control of neurotransmitter levels, and more particularly, being able to increase extracellular acetylcholine levels, particularly in nervous tissue.

Additionally, the primary neurotransmitter abnormalities rarely exist alone, and it is now well accepted that low acetylcholine levels accompanied by elevated glutamate play key roles in the development or worsening of various neurological dysfunctions.

Glutamate (Glu) is the main excitatory neurotransmitter in the nervous system, especially brain and spinal cord, of mammals wherein it is working at a variety of excitatory synapses playing thereby a central role in functions such as learning, pattern recognition, and memory (Bliss and Collingridge, Nature 361, 31-39, 1993). However, a large number of studies have furthermore established that cellular communication involving said excitatory amino acid can be transformed into a mechanism of cell destruction often referred to as excitotoxicity (Watkins and Collingridge, The NMDA receptor, IRL Oxford, 1989).

Normally extracellular levels of glutamate are elevated only in a brief and spatially localized fashion associated with normal synaptic transmission; however, glutamate can also be toxic to neurons *in vitro* and *in vivo* and it has been demonstrated that the function of glutamate receptors, especially glutamate receptors of the N-methyl-D-aspartate ("NMDA") receptor subtype, is crucial in a number of neuronal damages and injuries (Appel, Trends Neurosci.,16, 3-5, 1993). Many neurological disorders involving epileptic seizures and chronic or acute degenerative processes, such as for example Alzheimer's, Huntington's, Parkinson's diseases, multiple sclerosis (MS), amyotrophic lateral sclerosis (ALS), spinal muscular atrophy (SMA), retinopathy, stroke, depression and traumatic brain injury, involve neuronal cell death caused by over-stimulation of the glutamate receptors. These excessive activations of glutamate receptors, referred to as "glutamate excitotoxicity", are actually associated with abnormal elevation of extracellular glutamate levels. The mechanisms of the elevation of extracellular glutamate include enhanced efflux of glutamate and/or the reduction of glutamate uptake by cells. Moreover, it has been shown that said glutamate excitotoxicity may play a part in neurodegenerative diseases such as Alzheimer's disease and Huntington's disease.

Thus, according to a preferred embodiment of the present invention, it would be desirable to provide compounds which not only address the clinical aspects of neurotransmission dysfunctions, and more precisely the clinical aspects of acetylcholine neurotransmission dysfunctions, but which further protect cells, especially neurons, from glutamate-induced excitotoxicity , and more specifically which control not only the acetylcholine levels in the patient in need, but also the glutamate levels. Said special compounds will be referred in the followings as combination products.

For example, Ved et al. (1997, Neuroreport., 8, 963-968) have shown that Huperzine A, which is a therapeutic agent for Alzheimer's disease, inhibits acetylcholinesterase in primary cultures derived from forebrain, hippocampus, cortex and cerebellum of embryonic rat brain, and that pre-treatment of cell cultures with Huperzine A reduced cell toxicity, as evidenced by cytotoxicity assay and general morphology and glutamate-induced calcium mobilization. These authors conclude that Huperzine A could be a potent neuroprotective agent not only where cholinergic neurons are impaired, but also under conditions in which glutamatergic functions are compromised. Nevertheless, the authors conclude that said neuroprotective effect should result from antagonist effect of Huperzine A on NMDA receptor, hereby preventing its interaction with glutamate. No indication referring to the extracellular glutamate levels modulation by Huperzine A is provided.

The Inventors have now identified novel combination products, the various constituents of which are chosen so as to obtain a surprisingly synergistic effect of their respective regulatory activities towards control of neurotransmitter levels. More particularly, the combination products of the Invention make them possible to enhance extracellular acetylcholine levels and to decrease or to obviate glutamate levels extracellular increase. In particular the present invention pertains to the surprising discovery that NO or NO donor compounds and melatonin or melatonin derivatives, when combined, act synergistically to increase acetylcholine levels, with no or limited glutamate levels increase, or glutamate levels decrease. This absence of effect of the compounds, products or compositions of the Invention on glutamate levels, or their decreasing activity on glutamate levels, is generally referred to as "glutamate levels control" herein. These combined activities are particularly advantageous, as it allows maintaining neuron alive by limiting glutamate excitotoxicity, and thus extends the beneficial period of the treatment resulting from increasing the acetylcholine levels which is actually efficient only if neurons are present.

The gas NO is known as a messenger that modulates neuronal function. The use of NO donors and NO synthase inhibitors as pharmacological tools revealed that endogenous NO increases the release of several neurotransmitters, such as acetylcholine, catecholamines, excitatory and inhibitory amino acids, serotonin, histamine, and adenosine (for a review, refer to Prast and Philippu, 2001, Prog. Neurobiol., 64, 51-68). However, Lawrence and Jarrott (1993, Neurosci. Lett., 151, 126-129) have shown that NO donors like linsidomine, SNAP, hydroxylamine, NO gas additionally enhance the release of glutamate (see also Experimental Section). Similarly, Prast et al. (1998, J. Neurochem. 71, pp. 266-273) conclude that NO does not directly influence acetylcholine release from cholinergic neurons but actually acts on glutamate release upregulation which then play a key role for the NO-evoked enhancement of acetylcholine release. Thus, the observed glutamate levels control effect was not expected by using NO or NO donor compounds in the combination products of the Invention. Similarly, this synergistic result was totally unpredictable as recent published data have demonstrated that natural nocturnal pineal melatonin production was associated with an increase in glutamate release, and therefore of the extracellular glutamate levels (Markus et al., 2003, J. Pharmacol. Exp. Ther. available on January 24 2003 ; web reference 102045625-0). The present invention thus offers an advantageous, unexpected and effective alternative to the techniques of the prior art, in particular for treating and/or preventing various diseases and conditions mediated or associated by the imbalance of at least one of said two neurotransmitters in humans or in animals.

According to one embodiment of the present invention, the term "combination product" means that these two activities (i.e. acetylcholine levels enhancement and glutamate levels control) are characteristic of a single compound, as the one disclosed below. Alternatively, in another embodiment, "combination product" designates at least two separate compounds having independently from one another these two activities and which (i) are non combined or combined in a single mixture and (ii) are intended for an administration which is simultaneous (i.e. separate compounds are administered together), consecutive (i.e. separate compounds are administered separately but consecutively) or spread out over time (i.e. separate compounds are administered separately at different period of time).

First, the present invention concerns compounds of the general formula (I) or (II): 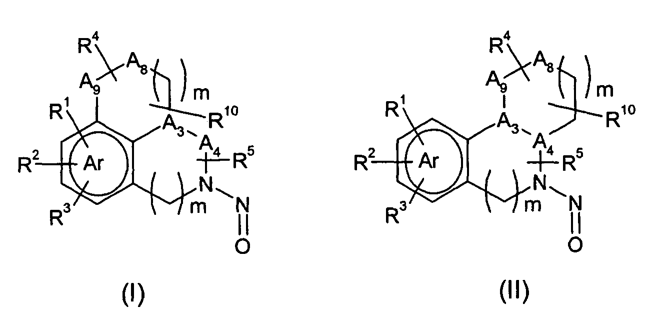 or analogues, derivatives, solvates or salts thereof,
wherein:
**R**^{**1**}**, R**^{**2**}**, R**^{**3**}**, R**^{**4**}**, R**^{**5**} **and R**^{**10**} are, independently from one another, H or a moiety of the following general formula : **-(R**^{**6**}**)**_{**n**}**-R**^{**7**}**;**
with **R**^{**6**}**, R**^{**6**}***** is a moiety selected in the group consisting of:
   (i) CH₂
   (ii) 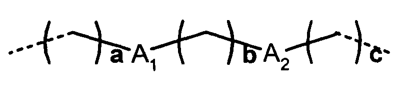
   (iii) 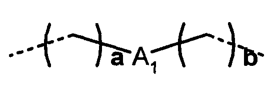 with:
      **a**, **b** and **c** are, independently from one another, an integer ranging from 0 to 4 ; **A**_{**1**} and **A**_{**2**} are, independently from one another, a moiety selected in the group consisting of -CO-, -CS-, -CN(Cₙ,H_{2n'+1})-, -O-, -NH-, -SO₂ - -C(C_{n'}H_{2n'+1})(C_{n'}H_{2n'+1}) -, -N(C_{n'}H_{2n'+1})-, -N(cycloalkyl)- [e.g. -N(cyclohexyl)-or N(phenyl)-];
**R**^{**7**} is, a moiety selected in the group consisting of -C_{n'}H_{2n'+1} , a cycloalkyl moiety [e.g. a cyclohexyl or a phenyl moiety], -N(C_{n'}H_{2n'+1})(C_{n'}H_{2n'+1}), -NH-cycloalkyl [e.g. -NH-cyclohexyl or -NH-phenyl], -O(C_{n'}H_{2n'+1}), -O-cycloalkyl [e.g. -0-cyclohexyl or -0-phenyl], =O, =S, -NO₂, , -I, -Br, -Cl, -F, -CF₃, -OCF₃, -COOH, -SO₃H, -PO₃H₂, -CN and 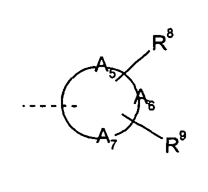 with:
   **R**^{**8**} and **R**^{**9**} are, independently from one another, a moiety of the following general formula: **-(R**^{**6***}**)**_{**n**}**R**^{**7***}
   **R**^{**6***}, independently from R⁶, being as defined above for R⁶, **R**^{**7***} is, a moiety selected in the group consisting of -Cₙ,H_{2n'}+₁ , a cycloalkyl moiety [e.g. a cyclohexyl or a phenyl moiety], -N(C_{n'}H_{2n'+1})(C_{n'}H_{2n'+1}), -NH-cycloalkyl [e.g. -NH-cyclohexyl or -NH-phenyl], -O(C_{n'}H_{2n'}+₁), -O-cycloalkyl [e.g. -0-cyclohexyl or -0-phenyl], =O, =S, -NO₂ -I, -Br, -Cl, -F, -CF₃, -OCF₃, -COOH, -SO₃H, -PO₃H₂, -CN;
   with in all the above:
   **A**_{**3**}**, A**_{**4**}**, A**_{**5**}**, A**_{**6**}**, A**_{**7**}**, A**_{**8**} and **A**_{**9**} are, independently from one another, an atom selected in the group consisting of C, N, O and S;
   **m** is an integer ranging from 0 to 2;
   **n** is, independently from one another, an integer ranging from 0 to 6;
   **n'** is, independently from one another, an integer ranging from 1 to 8, preferably from 1 to 4, preferably from 1 to 3 and more preferably from 1 to 2;
   **Ar** is a moiety selected in the group consisting of (a) a C₅ or C₆ aromatic ring, (b) an heteroaromatic ring comprising from 5 to 6 atoms and at least one heteroatom selected from N, O and S, (c) a C₈-C₁₂ bicyclic aromatic ring and (d) an heteroaromatic bicyclic ring comprising from 8 to 12 atoms and at least one heteroatom selected from N, O and S.

According to one embodiment, the present invention concerns compounds of the general formula (III): 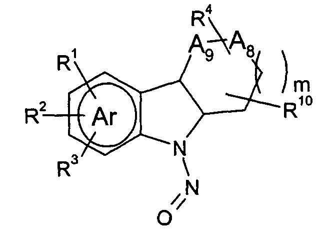 or analogues, derivatives, solvates or salts thereof,
wherein:
**R**^{**1**}**, R**^{**2**}**, R**^{**3**}**, R**^{**4**}**, R**^{**5**}**, R**^{**6**}**, R**^{**6***}**, R**^{**7**}**, R**^{**7**}***, R**^{**8**}**, R**^{**9**}**, R**^{**10**}**, A**_{**1**}**, A**_{**2**}**, A**_{**5**}**, A**_{**6**}**, A**_{**7**}**, A**_{**8**}**, A**_{**9**}**, a, b, c, m, n, n' and Ar being as above described.**

According to another embodiment, the present invention concerns compounds of the general formula (IV): 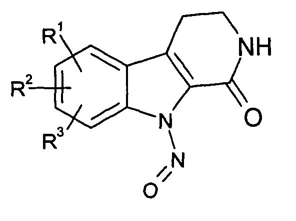 or analogues, derivatives, solvates or salts thereof,
**wherein:**
**R**^{**1**} **,R**^{**2**}**, R**^{**3**}**, R**^{**4**}**, R**^{**5**}**, R**^{**6**}**, R**^{**6***}**, R**^{**7**}**, R**^{**7***}**, R**^{**8**}**, R**^{**9**}**, A**_{**1**}**, A**_{**2**}**, A**_{**5**}**, A**_{**6**}**, A**_{**7**}**, a, b, c, n and n' being as above described.**

The invention also relates to compounds as described above of the general formula (V): 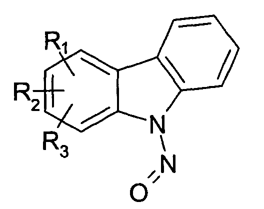 or analogues, derivatives, solvates or salts thereof,
wherein:
**R**^{**1**}**, R**^{**2**}**,** and **R**^{**3**}**,** being as above described.
The invention also relates more particularly to compounds as described above of the formula (Va): 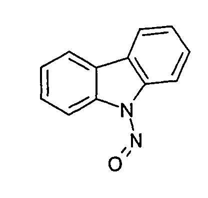 or analogues, derivatives, solvates or salts thereof.

According to special embodiments, when present, the moiety: 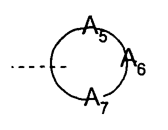 is intended to designate:
(i) a mono carbocyclic ring (i.e. a cyclic carboalkyl, with **A**_{**5**}, **A**_{**6**} and **A**_{**7**} are C);
(ii) a mono heterocyclic ring (i.e. a cyclic heteroalkyl, with at least one **A**_{**5**}**, A**_{**6**} and/or **A**_{**7**} is selected in the group consisting of N, S and O);
(iii) a bi-carbocyclic ring (i.e. a bicyclic carboalkyl with **A**_{**5**}**, A**_{**6**} and **A**_{**7**} are C);
(iv) a bi-heterocyclic ring (i.e. a bicyclic heteroalkyl with at least one cyclic ring is containing at least one **A**_{**5**}**, A**_{**6**} and/or **A**_{**7**} selected in the group consisting of N, S and O);
with said carbocyclic and/or heterocyclic ring (including both mono and bi) being unsaturated, partially or completely saturated, and is containing from 5 to 10 atoms.

According to special embodiments, when present, the moiety: 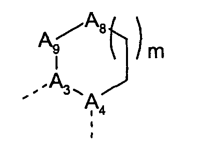 is intended to designate:
(i) a mono carbocyclic ring (i.e. a cyclic carboalkyl, with **A**_{**3**}**, A**_{**4**}**, A**_{**8**} and **A**_{**9**} are C);
(ii) a mono heterocyclic ring (i.e. a cyclic heteroalkyl, with at least one **A**_{**3**}, **A**_{**4**}**, A**_{**8**} and/or **A**_{**9**} is selected in the group consisting ofN, S and O);
(iii) a bi-carbocyclic ring (i.e. a bicyclic carboalkyl with **A**_{**3**}**, A**_{**4**}**, A**_{**8**} and **A**_{**9**} are C);
(iv) a bi-heterocyclic ring (i.e. a bicyclic heteroalkyl with at least one cyclic ring is containing at least one **A**_{**3**}**, A**_{**4**}**, A**_{**8**} and/or **A**_{**9**} selected in the group consisting ofN, S and O);
with said carbocyclic and/or heterocyclic ring (including both mono and bi) being unsaturated, partially or completely saturated, and is containing from 5 to 10 atoms.

According to special embodiments, when present, the moiety: 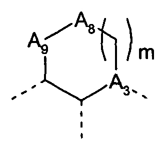 is intended to designate:
(i) a mono carbocyclic ring (i.e. a cyclic carboalkyl, with **A**_{**3**}, **A**_{**8**} and **A**_{**9**} are C);
(ii) a mono heterocyclic ring (i.e. a cyclic heteroalkyl, with at least one **A**_{**3**}**, A**_{**8**} and/or **A**_{**9**} is selected in the group consisting of N, S and O);
(iii) a bi-carbocyclic ring (i.e. a bicyclic carboalkyl with **A**_{**3**}, **A**_{**8**} and **A**_{**9**} are C);
(iv) a bi-heterocyclic ring (i.e. a bicyclic heteroalkyl with at least one cyclic ring is containing at least one **A**_{**3**}, **A**_{**8**} and/or **A**_{**9**} selected in the group consisting ofN, S and O);
with, said carbocyclic and/or heterocyclic ring (including both mono and bi) being unsaturated, partially or completely saturated, and is containing from 5 to 10 atoms.

According to special embodiments, the moiety: 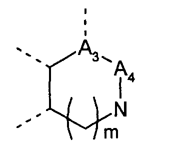 is intended to designate a mono heterocyclic ring (i.e. a cyclic heteroalkyl, with **A**_{**3**} and **A**_{**4**} selected in the group consisting of C, N, S and O) with said heterocyclic ring being unsaturated, partially or completely saturated, and is containing from 5 to 10 atoms.

According to one special embodiment, the cycles designated with: 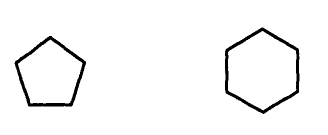 are under the form of an aromatic cycle :

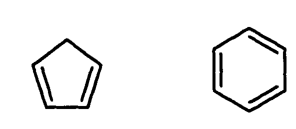

According to the present invention, the substituting moiety R present in the moiety: 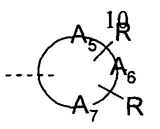 for example a cycle, such as for example the followings: 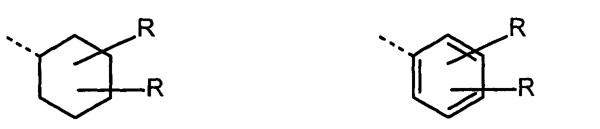 can be localized in position para, meta and/or ortho of said cycle.

Examples of said substitutions are:

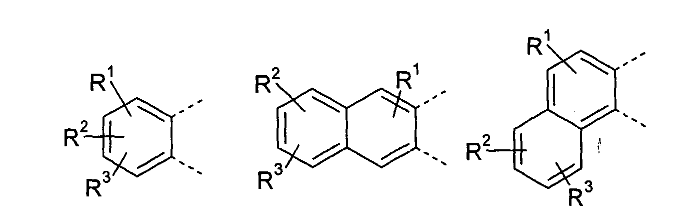

According to another special embodiment, at least one moiety selected in the group **R**^{**1**}**, R**^{**2**} and **R**^{**3**} is in position 7 in formula (II), (III), (IV), or (V) with:

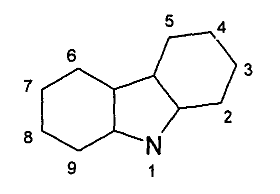

According to the present invention, the term "C_{n'}H_{2n'+1}" as used herein, alone or in combination, is intended to designate a straight or branched chain, or cyclic carbon radical, or combination thereof, which may be fully saturated, mono- or polyunsaturated and can include di- and multi-moities. Typically, an C_{n'}H_{2n'+1} moiety will have from 1 to 24 carbon atoms, with those moieties having 10 or fewer carbon atoms being preferred in the present invention. In rather preferred embodiment, the C_{n'}H_{2n'+1} moieties of the invention are alkyl. A "alkyl" is a shorter C_{n'}H_{2n'+1} chain having eight or fewer carbon atoms (e.g. n'≤ 8), preferably six or fewer carbon atoms (e.g. n'≤ 6), and even more preferably 4 or fewer carbon atoms (i.e. C₁₋₄). Typically, a C₁₋₄ alkyl moiety according to the invention will have from 1 to 2 carbon atoms. Examples of saturated alkyl moieties include, but are not limited to, methyl, ethyl, n-propyl, isopropyl, n-butyl, t-butyl, isobutyl, sec-butyl, tert-butyl, cyclopropyl, cyclobutyl, cyclopentyl, cyclohexyl, cycloheptyl and cyclooctyl, (cyclohexyl)methyl, cyclopropylmethyl, n-pentyl, isopentyl, n-hexyl, isohexyl, n-heptyl, isoheptyl, n-octyl, and the like. An unsaturated alkyl moiety is one comprising one or more double bonds or triple bonds. Examples of unsaturated alkyl moieties include, but are not limited to, aromatic cycles such as phenyl and benzyl.

Additionally, the term "alkyl" is intended to further include those derivatives of alkyl comprising at least one heteroatom, selected from the group consisting of O, N and/or S (i.e. at least one carbon atom is replaced with one heteroatom). These alkyl derivatives are widely named "heteroalkyl" and as alkyl above described are intended to designate, by themselves or as part of another substituent, stable straight or branched chains, or cyclic moieties, or combinations thereof. According to specific embodiment, the nitrogen and sulfur atoms when present in the said heteroalkyl are further oxidized and/or the nitrogen heteroatom is quaternized. The heteroatom may be placed at any position of the heteroalkyl moiety, including the position at which the alkyl moiety is attached to the remainder of the molecule.

The terms "cycloalkyl" and "heterocycloalkyl", by themselves or as part of another substituent, are intended to designate cyclic versions of the above "alkyl" and "heteroalkyl", respectively. They include bicyclic, tricyclic and polycyclic versions thereof.

It should be noted that the compounds of the Invention are comprising several moieties that can be repeated nn n' or m times; it should be understood that each n, n' or m values throughout the formula (I), (II), (III), (IV) or (V) in a particular compound can be chosen independently from one another. According to special embodiments of the invention, n is an integer ranging from 0 to 4, more particularly from 0 to 2, and even more particularly from 0 to 1. In a special case it is 0.

Similarly, the compounds of the Invention are comprising several moieties and/or atoms that can be present many times throughout one particular formula (I), (II), (III), (IV) or (V) (e.g. **A**_{**1**}**-A**_{**9**}**, R**^{**1**}**, R**^{**2**}**, R**^{**9**}**, R**^{**7**}**...);** it should be understood that each individual moieties and/or atoms throughout the formula of the Invention in a particular compound can be chosen independently from one another.

According to the present invention, the term C₁₋₄ alkyl is intended to designate a straight or branched chain, which may be fully saturated, mono- or polyunsaturated, having from 1 to 4 carbon atoms, such as methyl, ethyl, n-propyl, iso- propyl, and the like. Typically, a C₁₋₄ alkyl moiety according to the invention will have from 1 to 2 carbon atoms, with those moieties having 1 carbon atom being preferred in the present invention. An unsaturated alkyl moiety is one comprising one or more double bonds or triple bonds.

Alternatively, at least one moiety "C_{n'}H_{2n'+1}" in one of the compound of the invention can be substituted with "C_{n'}H_{2n'+1} moiety" derivative wherein at least one hydrogen atom is replaced with a fluorine atom.

The terms "analogues, derivatives, solvates or salts of compounds of the present invention" include both the structural derivatives and analogues of said compounds, their pharmaceutically acceptable solvates or salts, their stereoisomers, esters, prodrug forms, or their polymorphs. All these type of compounds are herein designated by the generic term "compounds".

Those skilled in the art will recognize that the compounds of the present invention may be utilized in the form of a pharmaceutically acceptable salt thereof. The physiologically acceptable salts of the compounds of the Invention include conventional salts prepared with pharmaceutically acceptable acids or bases, depending on the particular substituents found on the compounds described herein for dosing in mammals, especially humans. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, formic, monohydrogencarbonic, phosphoric, monohydrogenphosphoric, dihydrogenphosphoric, perchloric, sulfuric, monohydrogensulfuric, hydriodic, or phosphorous acids and the like, as well as the salts derived from organic acids like acetic, lactic, propionic, isobutyric, palmoic, maleic, glutamic, hydroxymaleic, malonic, benzoic, succinic, glycolic, suberic, fumaric, mandelic, phthalic, salicylic, benzenesulfonic, p-tolylsulfonic, citric, tartaric, methanesulfonic, hydroxynaphthoic, hydroiodic, and the like. Other acids such as oxalic, while not considered as pharmaceutically acceptable, may be useful in the preparation of salts useful as intermediates in obtaining the compounds of the invention and their pharmaceutically acceptable salts. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, lithium, calcium, aluminium, ammonium, barium, zinc, organic amino, or magnesium salt, N,N¹-dibenzylethylenediamine, choline, diethanolamine, ethylenediamine, N-methylglucamine, procaine salts (e.g. chloroprocaine) and the like. Also included are salts of amino acids such as arginate and the like, and salts of organic acids like glucuronic or galactunoric acids and the like (see, for example, Berge et al, "Pharmaceutical Salts", Journal of Pharmaceutical Science, 66, 1-19). Finally, certain specific compounds of the present invention contain both basic and acidic functionalities that allow the compounds to be converted into either base or acid addition salts.

Similarly, those skilled in the art will recognize that the compounds of the present invention may be utilized in the form of a pharmaceutically acceptable solvate thereof. These solvates may be prepared by conventional methods such as dissolving the compounds of the Invention in solvents such as methanol, ethanol and the like.

References hereinafter to a compound according to the invention include both compounds of Formula (I), (II), (III), (IV) or (V) and their pharmaceutically acceptable salts and solvates.

Additionally, those skilled in the art will recognize that stereocenters exist in compounds of the Invention. Accordingly, the present invention includes all possible stereoisomers including optical and geometric isomers of Formula (I). It further includes not only racemic compounds, or racemic mixtures thereof, but also the optically active isomers as well. When a compound of the Invention is desired as a single enantiomer, it may be obtained either by resolution of the final product or by a stereospecific synthesis from either optically pure starting material or any convenient intermediate. Additionally, in situations where tautomers of the compounds of the Invention are possible, the present invention is intended to include all tautomeric forms of the compounds. These terms and methods required for identifying and selecting the desired compounds are well known in the art. For example, diastereoisomers may be separated by physical separation methods such as fractional crystallization and chromatographic techniques, and enantiomers may be separated from each other by the selective crystallization of the diastereomeric salts with optically active acids or bases or by chiral chromatography. Pure stereoisomers may also be prepared synthetically from the appropriate stereochemically pure starting materials, or by using stereoselective reactions.

In special embodiments, e.g in the case of the -COOH being present, the compounds of the present invention might be in a prodrug form. A prodrug is in most cases a pharmacologically inactive derivative of a parent drug molecule that requires spontaneous or enzymatic transformation within the body in order to release the active drug, and that has improved delivery properties over the parent drug molecule. Therefore, prodrugs of a compound of general formula (I), (II), (III), (IV) or (V) is a compound which has chemically or metabolically cleavable groups and which readily undergoes chemical changes under physiological conditions to provide a compound of the *Invention in vivo.* Prodrugs include acid derivatives well known to practitioners of the art, such as, for example, alkyl esters prepared by reaction of the parent acid compound with a suitable alcohol, or amides prepared by reaction of the parent acid compound with a suitable amine. Particularly preferred alkyl esters as prodrugs are formed from methyl, ethyl, propyl, isopropyl, n-butyl, isobutyl, tert-butyl, morpholinoethyl, and N,N-diethylglycolamido. Methyl ester prodrugs, for example, may be prepared by reaction of the acid form of a compound of general formula (I), (II) or (III) in a medium such as methanol with an acid or base esterification catalyst (e.g., NaOH, H₂SO₄). Ethyl ester prodrugs are prepared in similar fashion using ethanol in place of methanol. Details regarding prodrugs are available for example in US 5,498,729.

Those skilled in the art are further able to prepare various polymorphs of a compound of general formula (I), (II), (III), (IV) or (V) for example by crystallization of compound of the Invention under different conditions. For example, he can use different solvents or mixtures commonly used for crystallization. Similarly, he can crystallize compounds of general formula (I) at different temperatures, according to various modes of cooling, ranging from very fast to very slow cooling during crystallizations. Polymorphs may also be obtained by heating or melting the compound followed by gradual or fast cooling. The presence of polymorphs may be determined by solid probe NMR spectroscopy, IR spectroscopy, differential scanning calorimetry, powder X-ray diffraction or such other techniques.

According to special embodiments, the compounds of the invention may be labeled in a variety of ways. For example, the compounds may contain radioactive isotopes such as, for example H³ (tritium), I¹²⁵, I¹²³, or C¹⁴ at one or more of the atoms that constitute compounds of general formula (I), (II), (III), (IV) or (V). Similarly, the compounds may be advantageously joined directly, covalently or noncovalently, or through a linker molecule, to a wide variety of other moieties, which may provide function as carriers, labels, adjuvents, coactivators, stabilizers, etc. Such labeled and joined compounds are contemplated within the present invention.

Examples of compounds of the Invention are:
6-Methoxy-9-nitroso-2,3,4,9-tetrahydro-beta-carbolin-1-one,
6-Methoxy-9-nitroso-2,9-dihydro-beta-carboline.,
9-Nitroso-9H-carbazole

According to another embodiment, the present invention concerns combination products as above defined. In one specific embodiment said combination product consists in a compound selected in the group consisting of compounds of general formulas (I), (II), (III), (IV) or (V). According to said embodiment, the combination product is a compound presenting itself both the acetylcholine levels enhancement and the glutamate levels control activities.

Alternatively, in another specific embodiment, said combination product consists in :
(i) NO or at least one NO donor compound, and
(ii) melatonin or at least one melatonin derivative compound.

According to one embodiment, the combination product of the invention consists in a mixture comprising both (i) and (ii).

According to another embodiment, the combination product of the invention consists in a kit of product comprising (i) and (ii) separately, i.e. not mixed.

As used herein, "NO" means invariably nitric oxide or nitrogen oxide. It refers to uncharged nitric oxide, as well as negatively charged nitric oxide (i.e. nitroxyl ion, NO-) and positively charged nitric oxide (i.e. nitrosonium ion, NO⁺). It can be provided by gaseous nitric oxide however because of the somewhat cumbersome nature of said delivery, preferably NO is provided indirectly by compounds which generate NO or related N-oxide species, directly or indirectly, i.e. "compounds able to release, induce and/or promote NO formation in cells". Examples of said compounds also widely called "NO donors" encompass precursor of nitric oxide, i.e. substrates of NO synthase (NOS) which are metabolized in cells and thus induce NO formation, such as for example L-Arginine (2-amino-5-guanidinovaleric acid) or its derivatives, e.g. hydroxy-arginine or its boron derivatives. Other ''NO donors" are well known by those skilled in the art (see for example Feelisch, 1998, Naunyn-Schiedeberg's Arch Pharmacol, 358, 113-122). As disclosed in said revue, known examples of NO donors are organic nitrates (RONO₂s) (e.g. glyceryl trinitrate, pentaerythrityl tetranitrate, isosorbide dinitrate or isosorbide 5-mononitrate), S-nitrosothiols (RSNOs) (e.g. S-nitrosoglutathione or GSNO, S-nitroso-N-acetyl-DL-penicillamine or SNAP and S-notrosoalbumin), linsidomine, hydroxylamine, sydnonimines (e.g. N-ethoxycarbonyl-3-morpholino-sydnonimine or molsidomine and 3-morpholino-sydnonimine or SIN-1), NONOates (e.g. 3-(2-hydroxy-2-nitroso-1-propylhydrazino)-1-propanamine or PAPA NONOate, ref. 82140, Cayman Chemical), sodium nitroprusside or potassium pentachloronotrosylruthenium.

As used herein, "melatonin" or N-acetyl, 5-methoxytryptamine refers to a hormone naturally secreted by the pineal gland. This compound is well known in the art and is widely commercially available (ref. 63610, Sigma-Aldrich).

"Melatonin derivative" refers to any substituted or modified melatonin, wherein said substitution or modification does not significantly affect its property towards acetylcholine and glutamate levels control, i.e. said substituted or melatonin derivative acts as do the melatonin when present in a combination product of the invention. Examples of melatonin derivatives are available, in Leclerc et al., 2002, J. Med. Chem ., 45, 1853-1859 ; Bonnefont-Rousselot et al., 2002, J . Pineal Res., 33, 109-117 (e.g. N-[2-(5-methoxy-1H-indol-3-yl)ethyl]-3,5-di-tert-butyl-4-hydroxybenzamide (DTBHB) and (R,S)-1-(3-methoxyphenyl)-2-propyl-1,2,3,4-tetrahydro-beta-carboline (GWC20)).

According to an alternative embodiment, the melatonin in (ii) of the combination product of the invention is replaced by serotonin. Serotonin is commercially available (e.g. Sigma-Aldrich ref. 85036).

The combination product according to the invention, especially when it is under the form of a kit of product, can be administered simultaneously or consecutively or so as to be staggered over time. "Simultaneously" refers to a co-administration. In this case, the two essential components (i) and (ii) can be mixed to form a composition prior to being administered, or can be administered at the same time to the patient in need thereof. It is also possible to administer them "consecutively", that is to say one after the other, irrespective of which component of the combination product according to the invention is administered first. Finally, it is possible to use a mode of administration which is "staggered over time" or is intermittent and which stops and restarts at intervals which may or may not be regular. The time interval between the administrations is not critical and can be defined by the skilled person. It is possible to recommend an interval of from 10 min to 72 h, advantageously of from 30 min to 48 h, preferably of from 1 to 24 h and, very preferably, of from 1 to 6 h. It is pointed out that the routes and sites of administration of the two components can be different (systemic delivery and targeted administration, for example). According to one particularly preferred embodiment, the (i) NO or at least one NO donor compound is mixed with (ii) melatonin or at least one melatonin derivative compound before administration.

The invention further concern composition comprising at least one compound of the general formulas (I), (II), (III), (IV) or (V) and/or combination products as above disclosed.

The compounds, combination products or compositions of the present invention have the ability to modulate neurotransmitters levels, and more particularly they have the ability to "act to increase acetylcholine extracellular levels". Said special property can be tested by routine *in vitro* methods by the one skilled in the art, for example in brain slices, cultured neurons, nerve ending preparations (i.e., synaptosomes) or in nerve tissue or organs preparation (see for example WO0113108 the full content of which is incorporated herein by reference). See also the Experimental Section. Generally, typical compounds, combination products or compositions useful in carrying out the present invention provide for the improvement of acetylcholine levels in amounts of at least one third, typically at least about 10 times more, frequently at least about 100 times more, and sometimes at least about 1,000 times more, than those measured in absence of the said compounds, combination products or compositions tested in identical conditions.

According to special embodiments, the compounds, combination products or compositions of the present invention have the ability to modulate neurotransmitters levels, and more particularly they have the ability to "act to control glutamate levels". Said special property can be tested by routine *in vitro* methods by the one skilled in the art, for example in nerve ending preparations (i.e., synaptosomes) or in nerve tissue preparation (see for example WO0113108 the full content of which is incorporated herein by reference). See also the Experimental Section. Generally, typical compounds, combination products or compositions useful in carrying out the present invention allow to control of extracellular glutamate levels in the sense that the glutamate levels measured in presence of the said compounds, combination products or compositions is not significantly different or is reduced compared to the one measured in absence of the said compounds, combination products or compositions in identical conditions. "Is not significantly different" means that the extracellular glutamate levels increase, if any, is less than about 20%, preferably less than 10%, advantageously less than 5%, and highly advantageously is about 0%.

According to one special embodiment, the compounds, combination products or compositions of the present invention "act to increase acetylcholine extracellular levels" without modifying the extracellular glutamate levels. In this special case, the compounds, combination products or compositions useful in carrying out the present invention :
(a) improve the acetylcholine levels in amounts of at least one third, typically at least about 10 times more, frequently at least about 100 times more, and sometimes at least about 1,000 times more, than those measured in absence of the said compounds, combination products or compositions tested in identical conditions, and
(b) do not significantly affect, neither by increasing nor by decreasing, extracellular glutamate levels tested in identical conditions in presence or absence of said compounds, combination products or compositions.

According to another special embodiment, the compounds, combination products or compositions of the present invention "act to increase acetylcholine extracellular levels" and "act to control extracellular glutamate levels", these terms being as above defined.

The compounds, combination products or compositions of the present invention can further be characterized by their properties leading to the observed increase of acetylcholine extracellular levels. More specifically, the Applicant has shown that said increase activity of the compounds of general formula (I), (II), (III), (IV) or (V) or combination products of the invention is, at least in part, related to an anticholinesterase activity. More specifically, the compounds or combination products of the Invention are, at least in part, an acetylcholinesterase and/or butyrylcholinesterase inhibitor, said inhibition activity being partial or total. The cholinesterase inhibiting activity of the active compounds of the invention may be determined by a number of standard biological or pharmacological tests. One such procedure for determining cholinesterase inhibition are widely disclosed and implemented in the art, see for examples, Ellman et al., 1961, Biochem. Pharm., 1, 88-95 or in US 6,326,139.

It is further possible to analyze the modulating properties of the compounds, combination products or compositions of the present *invention in vivo,* in established animal models. These models are well known in the art (e.g. transgenic animals see for example, Wong et al., 2002, Nat. Neurosci.,5, 633-9 ; Duff, 2001, Biochem. Soc. Symp., 67,195-202 ; Janus and Westaway, 2001, Physiol. Behav., 73, 873-86 ; Link, 2001, Mech. Ageing Dev., 122, 1639-49 or US 5,387,742 or US 6,374,130). It is further possible to analyze the modulating properties of the compounds, combination products or compositions of the present invention in *in vivo* test of memory using wild type animal model or specific models.

Another aspect of the present invention is a method for modulating the acetylcholine, and eventually the glutamate, levels in a cell, a tissue and/or a patient in need thereof. According to this method, the cell, tissue or patient is contacted with a sufficient concentration of at least one compound, combination product or composition of the Invention for an increase of extracellular acetylcholine levels, and eventually a control of the extracellular glutamate levels to be detected.

The compounds, combination products or compositions of the present invention due to their properties towards neurotransmitter levels, especially acetylcholine and/or glutamate levels, can serve as pharmaceuticals for controlling the biological effects of alteration in neurotransmitters levels, especially in the nervous tissues and the neurological dysfunctions produced thereby. More specifically they are capable of specifically increase the extracellular acetylcholine levels to reduce an associated pathology or provide or enhance a prophylaxis. Advantageously, they are further capable of specifically control (i.e. unchanged or decrease) the extracellular glutamate levels to reduce or obviate an associated pathology, or side effect, or provide or enhance a prophylaxis.

Accordingly, the present invention further concerns a composition comprising at least one compound or combination product of the invention as disclosed above and a pharmaceutically acceptable carrier or diluent. These pharmaceutical compositions may be prepared by conventional techniques, e.g. as described in Remington, 1995, The Science and Practise of Pharmacy, 19.sup.th Ed.

Typical compositions of the present invention comprise a pharmaceutically acceptable excipient which may be a carrier or a diluent or be diluted by a carrier, or enclosed within a carrier which can be in the form of a capsule, sachet, paper, tablets, aerosols, solutions, suspensions or other container. In making the combination products, conventional techniques for the preparation of pharmaceutical compositions may be used. For example, the active compounds will usually be mixed with a carrier or a diluent, or diluted by a carrier or a diluent, or enclosed within a carrier or a diluent which may be in the form of an ampoule, capsule, sachet, paper, tablets, aerosols, solutions, suspensions or other container. When the carrier serves as a diluent, it may be solid, semi-solid, or liquid material which acts as a vehicle, excipient, or medium for the active compound. The active compounds can be adsorbed on a granular solid container for example in a sachet. Typically, liquid oral pharmaceutical compositions are in the form of, for example, suspensions, elixirs and solutions; solid oral pharmaceutical compositions are in the form of, for example, powders, capsules, caplets, gelcaps and tablets. Because of their ease in administration, tablets and capsules represent the most advantageous oral dosage unit form, in which case solid pharmaceutical carriers are obviously employed.

Some examples of suitable carriers or diluents are, without being limited, water, salt solutions, alcohols, polyethylene glycols, polyhydroxyethoxylated castor oil, peanut oil, olive oil, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone.

Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art. In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. The compound of the present invention can also be administered in the form of liposome delivery systems, such as small unilamellar vesicles, large unilamellar vesicles, and multilamellar vesicles. Liposomes can be formed from a variety of lipids, including but not limited to amphipathic lipids such as phosphatidylcholines, sphingomyelins, phosphatidylethanolamines, phophatidylcholines, cardiolipins, phosphatidylserines, phosphatidylglycerols, phosphatidic acids, phosphatidylinositols, diacyl trimethylammonium propanes, diacyl dimethylammonium propanes, and stearylamine, neutral lipids such as triglycerides, and combinations thereof. They may either contain cholesterol or may be cholesterol-free. These can be prepared according to methods known to those skilled in the art, for example, as described in US 4,522,811. The pharmaceutical compositions of the invention can be sterilized and mixed, if desired, with auxiliary agents, emulsifiers, salt for influencing osmotic pressure, buffers and/or colouring substances and the like, which do not deleteriously react with the active compounds.

The pharmaceutical compositions of the invention will typically be those which contain an effective amount of a compound of the invention. In general, such compositions should contain at least 0.5% of active compound(s), but the concentration may vary depending upon the particular form and may be from 4 to 70 weight percent (based on the total weight of the unit). The oral dosage unit typically contains between 1.0 mg to 300 mg of active compound.

Likewise, the compositions of the present invention can further comprise additional agents. Examples of such additional agents are, for example, substrate for synthesis of acetylcholine (e.g., choline, CDP-choline, phosphatidyl choline,...), agents stimulating the production and secretion of acetylcholine (e.g., phosphatidyl serine, CDP-choline, huperzine A), inhibitors of acetylcholinesterase/ butyrylcholinesterase (AChE/BuChE) (e.g., tacrine (Cognex), E2020 (Aricept), donepezil, galanthamine (Reminyl), rivastigmine (Exelon), huperzine A), inhibitors of acetylcholine re-uptake , agents that facilitate improved binding at the receptor site (e.g., phosphatidyl serine and dioleylphosphatidic acid), agents mimicking the effects of acetylcholine by acting directly on, for example, nicotinic receptors (nicotinic agonists, e.g. ABT-594), and agents inducting enzymes used to synthesize acetylcholine (e.g., soy phytoestrogens). Literature provides to the skilled man with numerous examples of such additional agents.

The compounds, combination products or compositions of the present invention exhibit modulating activity toward neurotransmitters, and preferably towards acetylcholine and glutamate extracellular levels, which are important factors at the occurrence of neurological disorders.

Accordingly, the compounds, combination products or compositions of the present invention are specially adapted to cure, improve or prevent one or more symptoms of diseases or pathologic conditions associated with the imbalance of acetylcholine and are therefore particularly useful in the treatment and/or prevention, for example, of cognitive disorders and/or neurological dysfunction and/or mood disturbances such as, but not limited to, degenerative nervous system diseases or dementia. Thus, a further aspect of the present invention is a method for the treatment and/or prevention of diseases and conditions mediated by the imbalance of acetylcholine, the method comprising administering to the patient in need a therapeutically effective amount of at least one compound selected in the group consisting in compounds of Formula (I), (II), (III), (IV) or (V) or a pharmaceutically composition as above disclosed. Similarly, the present invention concerns a method for modifying the acetylcholine extracellular levels, and more specifically for increasing said levels, comprising administering to the patient in need a therapeutically effective amount of at least one combination products of the invention, or a pharmaceutically composition as above disclosed.

According to advantageous embodiment, a further aspect of the present invention is a method for the treatment and/or prevention of diseases and conditions mediated by the imbalance of acetylcholine, the method comprising administering to the patient in need a therapeutically effective amount of at least one combination products or a pharmaceutically composition as above disclosed, wherein said treatment is further associated with no increase (i.e. no change), and preferably with a reduction of the extracellular glutamate levels.

It will be appreciated by those skilled in the art that the term "treatment and/or prevention" herein extends to prophylaxis as well as the treatment of established diseases or symptoms.

"Diseases and conditions mediated by the imbalance of acetylcholine" refers to a variety of therapeutic applications, such as the treatment of neurodegenerative disorders such as Alzheimer's disease, and other disorders involving dysfunction of the central or autonomic nervous systems including memory loss and/or dementia ; disorders of extrapyramidal motor function such as Parkinson's disease, progressive supramuscular palsy, Huntington's disease, Gilles de la Tourette syndrome and tardive dyskinesia ; Parkinsonism-dementia-complex of Guam ; Dementia with Lewy bodies; Down's syndrome, Dementia pugilistica ; Pick's disease ; Post-alcoholic dementia ; Vascular dementia ; Creutzfeldt-Jakob's disease ; progressive supranuclear palsy; olivoponto-cerebellar atrophie ; subacute sclerosing panencephalitis with dementia ; cognitive disorders including disorders of attention, focus and concentration ; cognitive dysfunction ; mood and emotional disorders such as depression, panic, anxiety and psychosis ; neuroendocrine disorders; affective disorders ; substance abuse including withdrawal syndromes and substitution therapy; disorders and dysregulation of food intake, including bulemia and anorexia; disorders of nociception and control of pain; autonomic disorders including dysfunction of gastrointestinal motility and function such as inflammatory bowel disease, irritable bowel syndrome, diarrhea, constipation, gastric acid secretion and ulcers; pheochromocytoma; cardiovascular dysfunction including hypertension and cardiac arrhythmias, comedication in surgical procedures, and the like.

The Invention further concerns a method of alleviating or limiting the negative effects of a neurological disorder or neurodegenerative disease stemming from the aberrant synthesis, production, release or uptake or degradation of acetylcholine in a subject comprising administering to a patient suffering from said disorder or disease an effective amount of a compound, combination product or composition of the Invention.

According to one embodiment of the present invention, there is provided a method for treating and/or preventing diseases and conditions mediated by the imbalance of acetylcholine and glutamate, said method comprising administering to a patient in need of such treatment an amount of at least one combination product or a composition of the invention.

According to one embodiment of the present invention, there is provided a method for treating and/or preventing diseases and conditions mediated by the imbalance of acetylcholine and for treating and/or preventing glutamate excitotoxicity, said method comprising administering to a patient in need of such treatment an amount of at least one combination product or a composition comprising it of the invention. The neuroprotective effect of the combination products of the invention can be evaluated by known methods of the art, for example as disclosed in Ved et al, NeuroReport, 1997, 8, 963-968. Many neurological disorders involving epileptic seizures and chronic or acute degenerative processes, such as for example Alzheimer's disease, Huntington's disease, Parkinson's disease, AIDS-induced dementia, multiple sclerosis (MS), amyotrophic lateral sclerosis (ALS), spinal muscular atrophy (SMA), eye injuries, retinopathy, cerebral deficits due to cardiac bypass surgery and grafting, stroke, ischemia, hypoxia, hypoglycaemia, various traumatic brain injuries, drug-induced neurotoxicity, muscle spasms, convulsions, migraine, urinary incontinence, vomiting, dyskinesia, psychosis, schizophrenia, cognitive disorders and memory deficits, chronic and acute pain, anxiety, depression, which involve neuronal cell death caused by over-stimulation of the glutamate receptors.

According to one embodiment of the present invention, there is provided a method for improving memory capability, said method comprising administering to a patient in need of such treatment an amount of at least one combination product or a composition comprising it of the invention.

According to the present invention, the term "patient" means a mammal, e.g., a primate, e.g., a human.

By "pharmaceutically effective dose" is meant an amount of a pharmaceutical compound or composition having a therapeutically relevant effect in the frame of treatment and/or prevention of conditions mediated by the imbalance of acetylcholine. A therapeutically relevant effect relieves to some extent one or more symptoms of conditions mediated by the imbalance of acetylcholine, in a patient or returns to normal either partially or completely one or more physiological or biochemical parameters associated with or causative of said conditions. The compounds of the invention are effective over a wide dosage range. For example, in the treatment of adult humans, dosages from about 0.05 to about 100 mg, preferably from about 0.1 to about 100 mg, per day may be used. A most preferable dosage is about 0.1 mg to about 70 mg per day. In choosing a regimen for patients it may frequently be necessary to begin with a dosage of about 2 to about 70 mg per day and when the condition is under control to reduce the dosage as low as from about 0.1 to about 10 mg per day. The exact dosage will depend upon the mode of administration, on the therapeutic effect that is intended to be achieved, the form in which the dosage is administered, the subject to be treated and the body weight of the subject to be treated, and the preference and experience of the physician or veterinarian in charge. Dosages and treatment schedules are readily attainable by routine experimentation to those having ordinary skill in this art. Generally, the compounds are dispensed in unit dosage form comprising from about 0.1 to about 100 mg of active ingredient together with a pharmaceutically acceptable carrier per unit dosage.

The compounds or compositions of the present invention may be administered in a single daily dose, or the total daily dosage may be administered in divided doses of two, three or four times daily. Similarly, the treatment can be adapted to administer the compounds or compositions of the invention in a single weekly or monthly dose. Moreover, it will be appreciated that the amount of a compound of the invention required for use in treatment will vary with the nature of the condition being treated and the age and the condition of the patient and will be ultimately at the discretion of the attendant physician or veterinarian. In general, however, doses employed for adult human treatment will typically be in the range of 0.02 - 5000 mg per adult human per day, e.g., 1-1500 mg per adult human per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250 and 500 milligrams of the active ingredient for the symptomatic adjustment of the dosage to the patient to be treated. An effective amount of the drug is ordinarily supplied at a dosage level of from about 0.01 mg/kg to about 30 mg/kg of body weight per day. Particularly, the range is from about 0.03 to about 15 mg/kg of body weight per day, and more particularly, from about 0.05 to about 10 mg/kg of body weight per day. The compounds may be administered on a regimen of 1 to 2 times per day. Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

Toxicity and therapeutic efficacy of the compounds included in the compound or composition of the invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, special care should be taken to design a delivery system that targets such compounds to the site of affected tissue in order to minimize potential damage to uninfected cells and, thereby, leads to a reduction of side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The route of administration of the compound or composition of the present invention may be any route, which effectively transports the active compound to the appropriate or desired site of action, such as oral, nasal, pulmonary, transdermal or parenteral e.g. rectal, depot, subcutaneous, intravenous, intraurethral, intramuscular, intranasal, ophthalmic solution or an ointment, the oral or intratumoral route being preferred.

The present invention further concerns compounds and compositions of the present invention for use in therapy. Similarly, it concerns the use of at least one compound, combination product or composition according of the present invention for the manufacture of a medicament for the treatment of diseases and conditions where modification of the acetylcholine, and eventually glutamate, levels is of therapeutic benefit. Examples of these diseases and conditions are provided above.

Compounds of the general formula (I), (II), (III), (IV) or (V) above described can be prepared using readily available starting materials or known intermediates.

The compounds, combination products and compositions of the present invention may also find use in a variety *of in vitro* and *in vivo* assays, including diagnostic assays. In certain assays and in *in vivo* distribution studies, it is desirable to use labeled versions of the subject compounds, combination products and compositions, e.g. in radioligand displacement assays. Accordingly, the invention provides the compounds, combination products and compositions of the invention comprising a detectable label, which may be spectroscopic (e.g. fluorescent), radioactive, etc.

All publications and patent applications cited in this specification are herein incorporated by reference as if each individual publication or patent application were specifically and individually indicated to be incorporated by reference. Although the foregoing invention has been described in some detail by way of illustration and example for purposes of clarity of understanding, it will be readily apparent to those of ordinary skill in the light of the teachings of this invention that certain changes and modifications may be made thereto without departing from the spirit or scope of the appended claims.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practised otherwise than as specifically described. Accordingly, those skilled in the art will recognize, or able to ascertain using no more than routine experimentation, many equivalents to the specific embodiments of the invention described specifically herein. Such equivalents are intended to be encompassed in the scope of the following claims.

These and other embodiments are disclosed or are obvious from and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on Internet, e.g. under http://www.ncbi.nlm.nih.gov/PubMed/medline.html. Further databases and addresses, such as http://www.ncbi.nlm.nih.gov/,
http://www.infobiogen.fr/, http://www.fmi.ch/biology/research_tools.html,
http://www.tigr.org/, are known to the person skilled in the art and can also be obtained using, e.g., http://www.lycos.com. An overview of patent information in biotechnology and a survey of relevant sources of patent information useful for retrospective searching and for current awareness are given in Berks, TIBTECH 12 (1994), 352-364.

### Example:

### Synthesis of 9-Nitroso-9H-carbazole (FP 0140)

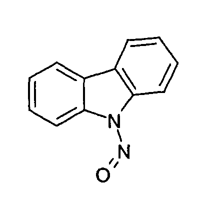

The expected product is obtained by nitrosation of commercial 9H-carbazole following the process described in Rinderknecht *et al (J. Org. Chem.,* 1953, 18, 971-982).. Yield: 47%. ¹H-NMR (300 MHz, D6-DMSO): 7.58-7.68 (m, 4H, Ar), 8.21-8.28 (m, 3H, Ar), 8.47-8.50 (m, 1H, Ar).

## Claims

1. Compounds of the general formula (I) or (II): 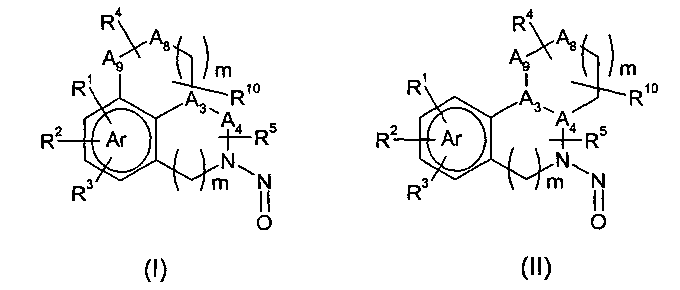 or analogues, derivatives, solvates or salts thereof,
wherein:
**R**^{**1**}**, R**^{**2**}**, R**^{**3**}**, R**^{**4**}**, R**^{**5**} and **R**^{**10**} are, independently from one another, H or a moiety of the following general formula : **-(R**^{**6**}**)**_{**n**}**-R**^{**7**}**;**
with **R**^{**6**} **, R**^{**6***} is a moiety selected in the group consisting of:
(i) CH₂
(ii) 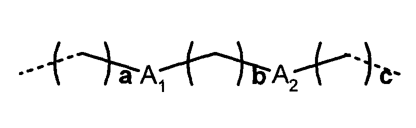
(iii) 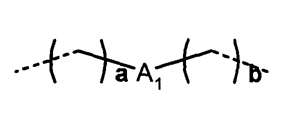 with:
**a, b** and **c** are, independently from one another, an integer ranging from 0 to 4 ;
**A**_{**1**} and **A**_{**2**} are, independently from one another, a moiety selected in the group consisting of -CO-, -CS-, -CN(C_{n'}H_{2n'+1})-, -O-, -NH-, -SO₂-, -C(C_{n'}H_{2n'+1})(C_{n'}H_{2n'+}1) -, -N(C_{n'}H_{2n'+1})-, -N(cycloalkyl)- [e.g. -N(cyclohexyl)-or -N(phenyl)-];
**R**^{**7**} is, a moiety selected in the group consisting of -C_{n'}H_{2n'+1}, a cycloalkyl moiety [e.g. a cyclohexyl or a phenyl moiety], -N(C_{n'}H_{2n'+1})(C_{n'}H_{2n'+1}), -NH-cycloalkyl [e.g. -NH-cyclohexyl or -NH-phenyl], -O(C_{n'}H_{2n'+1}), -O-cycloalkyl [e.g. -O-cyclohexyl or -O-phenyl], =O, =S, -NO₂, , -I, -Br, -Cl, -F, -CF₃, -OCF₃, -COOH, -SO₃H, -PO₃H₂, -CN and 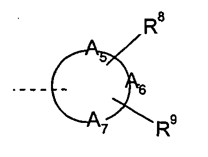 with:
**R**^{**8**} and **R**^{**9**} are, independently from one another, a moiety of the following general formula: **-(R**^{**6***}**)**_{**n**}**-R**^{**7***}
**R**^{**6***}**,** independently from R⁶, being as defined above for R⁶, **R**^{**7***} is a moiety selected in the group consisting of -C_{n'}H_{2n'+1}, a cycloalkyl moiety [e.g. a cyclohexyl or a phenyl moiety], -N(C_{n'}H_{2n'+1})(C_{n'}H_{2n'+1}), -NH-cycloalkyl [e.g. -NH-cyclohexyl or -NH-phenyl], -O(C_{n'}H_{2n'+1}), -O-cycloalkyl [e.g. -O-cyclohexyl or -O-phenyl], =O, =S, -NO₂, -I, -Br, -Cl, -F, -CF₃, -OCF₃, -COOH, -SO₃H, -PO₃H₂, -CN;
with in all the above:
**A**_{**3**}**, A**_{**4**}**, A**_{**5**}**, A**_{**6**}**, A**_{**7**}**, A**_{**8**} and **A**_{**9**} are, independently from one another, an atom selected in the group consisting of C, N, O and S;
**m** is an integer ranging from 0 to 2;
**n** is, independently from one another, an integer ranging from 0 to 6;
**n'** is, independently from one another, an integer ranging from 1 to 8, preferably from 1 to 4, preferably from 1 to 3 and more preferably from 1 to 2;
**Ar** is a moiety selected in the group consisting of (a) a C₅ or C₆ aromatic ring, (b) an heteroaromatic ring comprising from 5 to 6 atoms and at least one heteroatom selected from N, O and S, (c) a C₈-C₁₂ bicyclic aromatic ring and (d) an heteroaromatic bicyclic ring comprising from 8 to 12 atoms and at least one heteroatom selected from N, O and S.

2. Compounds according to claim 1 of the general formula (III): 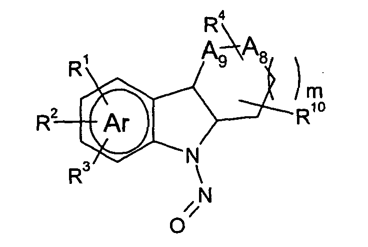 or analogues, derivatives, solvates or salts thereof,
wherein:
**R**^{**1**}**, R**^{**2**}**, R**^{**3**}**, R**^{**4**}**, R**^{**10**}**, A**_{**8**}**, A**_{**9**}**, n,** and Ar being as described in claim 1.

3. Compounds according to claim 1 or 2 of the general formula (IV): 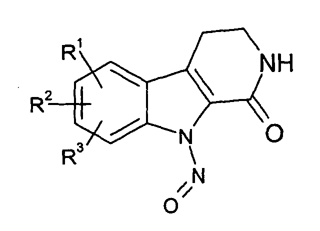 or analogues, derivatives, solvates or salts thereof,
wherein:
**R**^{**1**}**, R**^{**2**}**,** and **R**^{**3**}**,** being as described in claim 1.

4. Compounds according to claim 1 or 2 of the general formula (V): 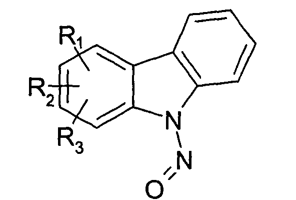 or analogues, derivatives, solvates or salts thereof,
wherein:
**R**^{**1**}**, R**^{**2**}**,** and **R**^{**3**}**,** being as described in claim 1.

5. Compounds according to claim 4 of the formula (Va): 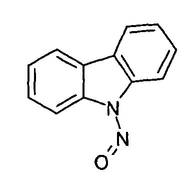 or analogues, derivatives, solvates or salts thereof.

6. A pharmaceutical composition comprising a pharmaceutically acceptable carrier and a pharmaceutically effective amount of at least one compound according to any one of claims 1 to 5, a pharmaceutically acceptable salt or a metabolically labile ester or amide thereof.

7. Use of at least one compound according to any one of claims 1 to 5, for the manufacture of a medicament for the treatment and/or prevention of diseases and conditions mediated by the imbalance of acetylcholine.

8. Use according to claim 7, of at least one compound according to any one of claims 1 to 5, for the manufacture of a medicament for the treatment and/or prevention of neurodegenerative disorders such as Alzheimer's disease, and other disorders involving dysfunction of the central or autonomic nervous systems including memory loss and/or dementia ; disorders of extrapyramidal motor function such as Parkinson's disease, progressive supramuscular palsy, Huntington's disease, Gilles de la Tourette syndrome and tardive dyskinesia ; Parkinsonism-dementia-complex of Guam ; Dementia with Lewy bodies; Down's syndrome, Dementia pugilistica ; Pick's disease ; Post-alcoholic dementia ; Vascular dementia ; Creutzfeldt-Jakob's disease ; progressive supranuclear palsy ; olivoponto-cerebellar atrophie ; subacute sclerosing panencephalitis with dementia ; cognitive disorders including disorders of attention, focus and concentration ; cognitive dysfunction ; mood and emotional disorders such as depression, panic, anxiety and psychosis ; neuroendocrine disorders; affective disorders ; substance abuse including withdrawal syndromes and substitution therapy; disorders and dysregulation of food intake, including bulemia and anorexia; disorders of nociception and control of pain; autonomic disorders including dysfunction of gastrointestinal motility and function such as inflammatory bowel disease, irritable bowel syndrome, diarrhea, constipation, gastric acid secretion and ulcers; pheochromocytoma; cardiovascular dysfunction including hypertension and cardiac arrhythmias, comedication in surgical procedures, and the like.

9. Use of at least one compound according to any one of claims 1 to 5, for the manufacture of a medicament for the treatment and/or prevention of diseases and conditions mediated by the imbalance of acetylcholine, and for treating and/or preventing glutamate excitotoxicity.

10. Use according to claim 9, of at least one compound according to any one of claims 1 to 5, for the manufacture of a medicament for the treatment and/or prevention of neurological disorders involving epileptic seizures and chronic or acute degenerative processes, such as Alzheimer's disease, Huntington's disease, Parkinson's disease, AIDS-induced dementia, multiple sclerosis (MS), amyotrophic lateral sclerosis (ALS), spinal muscular atrophy (SMA), eye injuries, retinopathy, cerebral deficits due to cardiac bypass surgery and grafting, stroke, ischemia, hypoxia, hypoglycaemia, various traumatic brain injuries, drug-induced neurotoxicity, muscle spasms, convulsions, migraine, urinary incontinence, vomiting, dyskinesia, psychosis, schizophrenia, cognitive disorders and memory deficits, chronic and acute pain, anxiety, depression.

11. Use of at least one compound according to any one of claims 1 to 5, for the manufacture of a medicament for improving memory capability.
